# EUROPÄISCHE PATENTSCHRIFT

(11) **EP 3 622 527 B1**
(45) Veröffentlichungstag und Bekanntmachung des Hinweises auf die Patenterteilung: **02.04.2025**
(21) Anmeldenummer: 18740092.4
(22) Anmeldetag: 09.05.2018
(51) Int. Cl.: G16H 40/00

(54) **SYSTEM ZUM ORTEN, SICHTEN, KLASSIFIZIEREN, VERWALTEN UND KOORDINIEREN BEI SCHADENSEREIGNISSEN**
SYSTEM FOR LOCATING, TRIAGING, CLASSIFYING, MANAGING AND COORDINATING DURING ACCIDENTS
SYSTÈME DE LOCALISATION, DE CONTRÔLE, DE CLASSEMENT, DE GESTION ET DE COORDINATION EN CAS D'ÉVÉNEMENTS DE DOMMAGES

(30) Priorität: 09.05.2017 DE 102017110014
(43) Veröffentlichungstag der Anmeldung: 18.03.2020
(73) Patentinhaber: ITK Engineering GmbH, 76761 Rülzheim (DE); Vomatec EDV GmbH, 55543 Bad Kreuznach (DE); 3i Ingenieure für Innovationsberatung und Informationsmanagement, 50679 Köln (DE)
(72) Erfinder: BREYER, Erich Matthias, 55435 Gau-Algesheim (DE); KASTER, Martina, 55442 Stromberg (DE); HEUER, Stephan, 76227 Karlsruhe (DE); DE KLERK, Richard, 76187 Karlsruhe (DE); WEBER, Benedikt, 50676 Pulheim (DE); KÄSER, Benjamin, 51069 Köln (DE); SCHÜTTE, Frederik, 50679 Köln (DE)
(74) Vertreter: Dr. Langfinger & Partner
(86) Internationale Anmeldenummer: PCT/DE2018/100451
(87) Internationale Veröffentlichungsnummer: WO 2018/206058

(56) Entgegenhaltungen:
- EP-A1- 2 752 813
- WO-A1-2008/146324
- JP-A- 2009 157 519
- JP-B2- 6 009 904
- US-A1- 2013 253 284
- ANONYMOUS: "Long Range Wide Area Network -", WIKIPEDIA, 19 April 2017 (2017-04-19), XP055835653, Retrieved from the Internet <URL:https://de.wikipedia.org/w/index.php?title=Long_Range_Wide_Area_Network&oldid=164710175> [retrieved on 20210827]
- JOSHI JETENDRA ET AL: "Health Monitoring Using Wearable Sensor and Cloud Computing", 2016 INTERNATIONAL CONFERENCE ON CYBERNETICS, ROBOTICS AND CONTROL (CRC), IEEE, 19 August 2016 (2016-08-19), pages 104 - 108, XP033042212, DOI: 10.1109/CRC.2016.031
- PALLAVI SETHI ET AL: "Internet of Things: Architectures, Protocols, and Applications", JOURNAL OF ELECTRICAL AND COMPUTER ENGINEERING, vol. 2017, no. ID9324035, 26 January 2017 (2017-01-26), pages 1 - 26, XP055633432, ISSN: 2090-0147, DOI: 10.1155/2017/9324035
- ANONYMOUS: "LoRaWAN | RS Components", WEBAUFTRITT, 18 May 2016 (2016-05-18), pages 1 - 4, XP093122338, Retrieved from the Internet <URL:https://web.archive.org/web/20160518024452/https://de.rs-online.com/web/generalDisplay.html?id=i/lora> [retrieved on 20240122]
- ANONYMOUS: "Simple triage and rapid treatment", WIKIPEDIA, 2 February 2017 (2017-02-02), XP055508177, Retrieved from the Internet <URL:https://en.wikipedia.org/w/index.php?title=Simple_triage_and_rapid_treatment&oldid=763223796> [retrieved on 20180919]

## Beschreibung

Die vorliegende Ertindung betrifft ein System zum Orten, Sichten, Verwalten und Organisieren bei Schadensereignissen.

Ein Massenanfall von Verletzten (MANV) bezeichnet eine Situation, bei der die Anzahl von zu versorgenden Personen die Versorgungskapazitäten übersteigt. Je nach Ausmaß und Umfang des Massenanfalls variiert der Bedarf an Einsatzmitteln beträchtlich.

Zu versorgende Personen sind in das Schadensereignis direkt oder indirekt involvierte Personen, die medizinisch, technisch, organisatorisch oder psychisch betreut werden müssen.

Versorgungskapazitäten subsummiert Organisationen, Menschen, Technik, die zur Bewältigung der Schadenslage berufen, bemächtigt oder einbezogen sind.

Dabei ist das erste Ziel der Bewältigung eines Massenanfalls von Verletzten eine möglichst rasche Wiederherstellung einer adäquaten Versorgungslage. Hierfür werden Einsatzmittel nicht nur aus der von dem Massenanfall von Verletzten betroffenen Region, sondern auch von Nachbarregionen alarmiert, und eine sogenannte erweiterte Führungsstruktur geschaffen, die die Einsatzmittel koordiniert.

Dabei sind wichtige Führungsfragen zu beantworten, beispielsweise wie die genaue Lagesituation ist, wie die (verletzten) Personen örtlich verteilt sind, welche Verletzungsmuster und -grade die Personen aufweisen, wie viele Personen bereits gesichtet wurden, wie viele Personen noch nicht gesichtet sind, wie viel Einsatzkräfte benötigt werden, usw.

Diese Führungsfragen sind in einer Situation erhöhter Unsicherheit und unter Zeitdruck zu beantworten, da meist ein Mangel an verfügbaren Ressourcen vorliegt, belastbare Informationen über die Lage fehlen und mit dem Zeitverlauf sich die Schadensauswirkung, z.B. bei den (verletzten) Personen, negativ entwickelt.

Hierbei kommt eine sogenannte Sichtung oder Triage zum Einsatz, ein nicht gesetzlich kodifiziertes Verfahren zur Priorisierung medizinischer Hilfeleistungen. Ohne eine strukturierte Triage besteht die Gefahr einer unethischen Selektion der betroffenen Personen. Zunächst kann eine Pre-Triage, auch Vorsichtung genannt, erfolgen, die eine schnelle Übersicht über die zu versorgenden Personen bieten soll.

Die Einschätzung der Erstsichter in der Pre-Triage entscheidet, welche Person zuerst versorgt werden soll. Als Richtzeit gilt dabei eine Zeit von 20 bis 60 Sekunden pro Person. Eine aufwändige Dokumentation soll dabei nicht erfolgen, vielmehr erfolgt eine Farbcodierung der Personen und es wird eine Strichliste der Personen und eine grobe Lageskizze erstellt.

Die Personen werden dabei anhand verschiedener möglicher Sichtungsalgorithmen in Sichtungskategorien eingeteilt. Weit verbreitet ist bspw. das sogenannte "Simple Triage and Rapid Treatment Schema" (STaRT-Schema), bei dem vier Kategorien zur Auswahl stehen: 1. T3/MINOR, Personen die selbst laufen können; 2. T1/IMMEDIATE, Personen im lebensbedrohlichen Zustand; 3. T2/DELAYED, alle anderen Personen; und T4/DECEASED, Personen die verstorben sind.

Alternativ zum STaRT-Schema sind zudem das modifizierte mSTaRT, das BASIC-Schema und das Reverse Triage Verfahren bekannt. Im Ausland sind ggf. weitere Verfahren bekannt. Alternative Verfahren werden ggf. in der notärztlichen Diskussion entwickelt bzw. bestehende Verfahren weiterentwickelt.

Nachteilig an den aus dem Stand der Technik bekannten Verfahren ist, dass der Zustand der Personen mittels Verletztenanhängekarten, auch als Triagekarten bezeichnet, dokumentiert wird und der Zustand der Personen per Funk oder direkte Meldung an eine Einsatzleitstelle übermittelt wird.

Dieser Vorgang ist zeitaufwändig, ungenau und fehlerträchtig. Zudem sind die Verletztenanhängekarten, auf denen der Zustand der Person üblicherweise mit einer Farbcodierung festgehalten wird, wobei jede Farbe für eine der Sichtungskategorien steht, in der Dunkelheit bzw. bei Regen nur schwer erkennbar. Auch können Personen von auf die Erstsichter nachfolgenden Einsatzkräften übersehen werden. Zudem ist die notwendige Dokumentation für die Sichtenden zeitaufwändig und hält diese von der weiteren Suche nach Personen ab. Weiterhin kann die Pre-Triage als auch die Triage in Papierform unbeabsichtigt sowie beabsichtigt verfälscht werden, da die farbigen Markierungsblätter verrutschen bzw. in ihren Initialzustand rutschen können.

Es wäre daher wünschenswert, eine Vorrichtung zu liefern, die eine schnellere und einfachere Sichtung ermöglichen würde.

Aus DE 10 2005 004 773 A1 ist ein Verfahren bekannt, dass die Sichtung vereinfachen soll. Hierbei wird eine Vorrichtung bereitgestellt, die an der Person angeordnet wird und die es ermöglicht, den Zustand der Person einzugeben, wobei der eingegebene Zustand sowie ein mittels GPS erfasster Ort der Person über WLAN an einen zentralen Server übermittelt werden.

Problematisch an dieser aus dem Stand der Technik bekannten Lösung ist zum einen, dass das aus dem Stand der Technik bekannte System nur sehr eingeschränkte Funktionalitäten aufweist und insbesondere keine ausreichende Übersicht über die Schadenslage bietet.

Des Weiteren ist es problematisch, dass WLAN oder Mobilfunkverbindungen oftmals im Falle eines Massenanfalls von Verletzen nicht zur Verfügung stehen oder überlastet sind.

Eine weitere alternative Lösung ist aus US 2013/ 0253284 A1 bekannt, bei der auf eine Sichtungsvorrichtung zurückgegriffen wird, die keine Batterie umfasst, aber einen Solid-State Speicher sowie eine Vielzahl von Aktuatoren. Jeder der Aktuatoren steht dabei für einen Zustand einer Person oder eine Logistikinformation. Um die Daten aus dem Solid-State Speicher auszulesen ist eine weitere Vorrichtung notwendig. Diese Datenauslese- und Übertragungsvorrichtung, insbesondere ein Mobiltelefon, soll dann in Interaktion mit einem Server zu stehen.

Diese Lösung ermöglicht somit ein Einstellen von Patientendaten mit einfachen Mitteln, indem Taster oder Schieberegler bedient werden, wobei diese analogen Daten in einem digitalen Speicher zusätzlich gespeichert werden können. Ein Auslesen des digitalen Speichers erfolgt dabei mittels einer weiteren Vorrichtung, da die Sichtungsvorrichtung selbst keine Batterie aufweist und keine Daten senden kann.

Der Vorteil dieses Systems, wie auch anderer vergleichbarer Systeme, bei denen entsprechende Daten in Daten-Tags, wie z.B. QR-Codes, hinterlegt werden, soll gemäß der dortigen erfindungsgemäßen Lehre darin bestehen, dass die Sichtungsvorrichtungen selbst bzw. die Daten-Tags sehr einfach und kostengünstig herzustellen sind und somit in Massen vorrätig sein können.

Nicht möglich wird es mit einer solchen Vorrichtung, dass Echtzeitdaten über Personen erfasst werden, sondern es wird nur ermöglicht, Informationen über einen Benutzer lokal analog und digital gleichzeitig zu speichern.

Die Aufgabe der vorliegenden Erfindung liegt nunmehr darin, die Nachteile des Standes der Technik zu überwinden und insbesondere ein System zu liefern, das eine schnelle und präzise Übersicht über die Anzahl der betroffenen Personen im Falle eines Massenanfalls von Verletzten in Echtzeit ermöglicht und eine Allokation der begrenzten Ressourcen optimiert.

Diese Aufgabe wird gelöst durch ein System zum Orten, Sichten, Klassifizieren, Verwalten und Koordinieren bei Schadensereignissen gemäß Anspruch 1.

Der Erfindung liegt dabei die überraschende Erkenntnis zugrunde, dass die Nachteile des Stands der Technik dadurch überwunden werden können, dass ein System bereitgestellt wird, das Sichtungsvorrichtungen umfasst, die an einer Person angeordnet werden können, wobei mittels der Sichtungsvorrichtungen der Zustand einer Person an einen oder mehrere zentrale Server übertragbar ist.

Dadurch wird es ermöglicht, dass alle relevanten Information auf dem mindestens einen Server aggregiert werden und in einer automatisierten Übersicht anzeigbar sind. Dies ermöglicht einen schnellen Überblick über die Schadenslage.

Es ist dabei insbesondere vorgesehen, dass die Sichtungsvorrichtung auch die Datenübertragungseinrichtung an den Server direkt umfasst und nicht eine getrennte Sichtungsvorrichtung und eine getrennte Datenübertragungseinrichtung vorliegen. Hierfür umfasst die erfindungsgemäße Sichtungsvorrichtung insbesondere eine Batterie bzw. einen Akkumulator, einen Mikroprozessor und eine Datenübertragungseinrichtung zur Kommunikation mit dem Server.

Gemäß dem Stand der Technik sind Lösungen beschrieben, bei denen die Sichtungsvorrichtung, die an einer Person angeordnet wird, getrennt von der Datenübertragungseinrichtung ausgebildet ist. Dies führt dazu, dass die Daten der Sichtungsvorrichtung nicht kontinuierlich zur Verfügung stehen, sondern nur, wenn sich die Datenübertragungseinrichtung zufälligerweise in der Nähe zu der jeweiligen Sichtungsvorrichtung befindet.

Ein erfindungsgemäßes System ermöglicht demgegenüber in vorteilhafter Weise, dass stets ein aktuelles Lagebild bereitgestellt werden kann, da die Sichtungsvorrichtungen die Datenübertragungseinrichtungen jeweils direkt mit umfassen. Ein solches stets aktuelles Lagebild ist vorteilhaft und erfindungswesentlich.

Dabei ist erfindungsgemäß vorgesehen, dass die drahtlose Kommunikation der Datenübertragungseinrichtung mit der Empfangseinrichtung über ein lokales Funknetz, eine Long range, low power wireless platform, ein Long Range Wide Area Network, SigFOX, WLAN, GSM, LTE und/oder UMTS erfolgt, wobei insbesondere die Sichtungsvorrichtungen mit Gateways kommunizieren und die Gateways die Datenpakete an den mindestens einen Server senden.

Die Datenübertragungseinrichtung kann mit der Empfangseinrichtung über verschiedene Datenübertragungsprotokolle kommunizieren. Neben den weit verbreiteten Möglichkeiten einer Datenübertragung über WLAN oder Mobilfunknetze hat es sich jedoch erfindungsgemäß als besonders vorteilhaft erwiesen, wenn eine Datenübertragung über ein lokales Funknetz, insbesondere über eine Long range, low power wireless platform und/oder ein Long Range Wide Area Network erfolgt.

Während im Falle eines Massenanfalls von Verletzten die Mobilfunknetze ausfallen können oder aufgrund von Überlastung zusammenbrechen können und WLAN gerade bei unebenem Gelände oder innerhalb von Gebäuden nur eine sehr begrenzte Reichweite aufweist, ermöglicht eine Long range, low power wireless platform und/oder ein Long Range Wide Area Network hohe Reichweiten von bis zu mehr als 10 km für die Uplink-Kommunikation. Die Netzarchitektur einer Long range, low power wireless platform bzw. eines Long Range Wide Area Network ist dabei sternförmig und die Endgeräte kommunizieren mit Gateways als Empfangseinrichtungen, welche die Datenpakete an einen Netzwerkserver weiterleiten.

Eine Verbindung der Gateways der Long range, low power wireless platform bzw. des Long Range Wide Area Network mit den Netzwerkservern kann dabei erfindungsgemäß bevorzugt mittels WLAN erfolgen, um einen schnellen Aufbau der Long range, low power wireless platform bzw. des Long Range Wide Area Network zu ermöglichen. Ein Long Range Wide Area Network nutzt dabei regional unterschiedliche Frequenzbereich des ISM-Bands, die üblicherweise im Bereich zwischen 433 MHz und 915 MHz liegen, und somit deutlich unter der Frequenz eines WLANs.

Eine Long range, low power wireless platform bzw. ein Long Range Wide Area Network kann dabei im Falle eines Massenanfalls von Verletzen schnell aufgebaut werden und je nach Anzahl der Gateways an unterschiedliche räumliche Begebenheiten angepasst werden, so dass eine optimale Abdeckung des Einsatzgebiets möglich ist.

Auch kann erfindungsgemäß bevorzugt sein, dass das System mindestens zwei Server umfasst, wobei sich die Server miteinander synchronisieren, so dass insbesondere ein redundantes System ausgebildet ist.

Das System kann dabei andere bereits vor Ort arbeitende Systeme erkennen und sich mit diesen automatisch vernetzen. Die Vernetzung ist dabei erfindungsgemäß unabhängig vom dem gewählten Datenübertragungsprotokoll der Kommunikation zwischen den Sichtungsvorrichtungen und dem Empfänger möglich, kann also über UMTS, WLAN oder das Funknetz der Sichtungsvorrichtungen stattfinden. Somit können auch große Einsatzgebiete abgedeckt werden.

Dabei kann insbesondere vorgesehen sein, dass mindestens einer der mindestens zwei Server in dem System anmeldbar oder abmeldbar ist
und/oder die Hierarchie der Server dynamisch festgelegt bzw. festlegbar ist, wobei die erfassten Daten zwischen den Servern automatisiert synchronisiert sind.

Dies hat insbesondere den Vorteil, dass sich die Daten zwischen allen Servern automatisiert synchronisieren. Bevorzugt ist das erfindungsgemäße System dabei ausgelegt und eingerichtet, um auch alternative Systeme vor Ort zu erkennen und sich mit diesen zu vernetzen. Dadurch entsteht automatisch eine Redundanz, wobei vorhandene Sichtungsvorrichtungen aufgrund der individuellen Kennung nicht doppelt angezeigt werden.

Das erfindungsgemäße System kann auf diese Weise beliebige Organisationshierarchien und Einsatzstrukturen unterstützen, auch wenn diese sich dynamisch ändern. Der Nutzer muss dazu nur in der Software seine Einsatzhierarchie aufbauen. Ein Wechsel der Einsatzleitung von einem Fahrzeug/Server auf ein anderes ist so einfach möglich, da die Daten zwischen den Fahrzeugen synchron gehalten werden.

Gemäß einer Ausführungsform der Erfindung kann dabei vorgesehen sein, dass jeder Zustand jedes Sichtungsgeräts eine von den anderen Zuständen unterscheidbare grafische Kennzeichnung aufweist, wobei insbesondere das Einstellelement in Form eines Dreh- und/oder Schiebeschalters ausgebildet ist, und vorzugsweise jeder Zustand des Dreh- oder Schiebschalters eine fest aufgebrachte farbliehe Markierung anzeigt.

Es wird somit ermöglicht, dass der Zustand jeder begutachteten Person ohne zusätzliche Hilfsmittel von den auf die Erstsichter nachfolgenden Einsatzkräften direkt erfasst werden kann, und die Anzahl der Personen für eine Planung der Anforderung weiterer Einsatzkräfte, Rettungswagen, Krankenhausbetten, etc. ohne zusätzlichen manuellen Übermittlungsaufwand bereitgestellt wird.

Die Ersthelfer sind bereits mit dem System der Verletztenanhängekarten vertraut und die Farbcodierung ist geläufig. Alternativ kann auch ein Folientaster zum Einsatz kommen, der jedoch im Vergleich zu den Dreh- und/ oder Schiebeschaltern nicht ohne eine Energieversorgung betrieben werden kann.

Durch eine fest aufgebrachte farbliche Markierung je nach Stellung des Einstellelements wird zudem ermöglicht, dass selbst im Falle einer technischen Fehlfunktion der erfindungsgemäßen Sichtungsvorrichtung sichergestellt ist, dass mindestens die gleiche Funktionalität der bisher verwendeten Verletztenanhängekarten aufrechterhalten wird.

Des Weiteren kann es vorteilhaft sein, dass die grafische Kennzeichnung in Form einer farblichen Markierung bereitgestellt ist, wobei der mittels des Einstellelements eingestellte Zustand mittels eines Anzeigeelements, insbesondere in Form von farblichen LEDs oder OLEDs, angezeigt ist.

Zusätzlich oder alternativ zu der festen farblichen Markierung am Einstellelement kann die erfindungsgemäße Sichtungsvorrichtung eine Anzeige des eingestellten Zustands der Person mittels eines Anzeigeelements umfassen. Dies hat insbesondere den Vorteil, beispielsweise bei Verwendung der als besonders bevorzugt angesehenen LEDs als Anzeigeelement, dass auch bei schlechten Licht- oder Wetterverhältnissen der Sichtungszustand einer Person einfach erkennbar ist. Zudem kann die Anzeigeeinrichtung der Lokalisierung von Personen dienen, da diese durch das Leuchten des Anzeigeelements schneller auffindbar sind. OLEDs haben dabei insbesondere den Vorteil, dass diese auch bei einer, ggf. zwischenzeitlichen, Unterbrechung der Energieversorgung einsatzbereit sind.

Gemäß der vorliegenden Erfindung ist das Einstellelement in Form eines Drehschalters und eines zusätzlichen Schiebeschalters ausgebildet, wobei der Drehschalter für jeden Zustand eine von den anderen Zuständen unterscheidbare grafische Kennzeichnung aufweist und durch den Schiebeschalter eine, insbesondere zweistufige, Priorisierung des mit dem Drehschalter eingestellten Zustands einstellbar ist.

Eine solche Kombination von Dreh- und Schiebeschalter hat den Vorteil, dass eine weiter untergliederte Priorisierung vorgenommen werden kann, indem beispielsweise die Kategorie T1/IMMEDIATE gemäß STaRT mittels des Drehschalters eingestellt wird, und besonders kritische Patienten über den Schiebeschalter hervorgehoben werden. Der Schiebeschalter umfasst dabei bevorzugt zwei Stellungen, eine Ausgangsposition und eine Stellung für erhöhte Priorität.

Des Weiteren kann es bevorzugt sein, dass mindestens eine Identifikationsvorrichtung umfasst ist, so dass eine Person eindeutig identifiziert oder identifizierbar ist, wobei die Identifikationsvorrichtung eine weitere Datenübertragungseinrichtung umfasst, um mit der Empfangseinrichtung zu kommunizieren.

Der Einsatz der erfindungsgemäßen Identifikationsvorrichtungen hat dabei insbesondere den Vorteil, dass serverseitig nicht nur verletzte Personen automatisch erfasst werden können, sondern parallel auch die Position und die Berufsgruppe der Rettungskräfte erkannt wird. Dies ist im Rahmen der Ressourcenallokation von großer Bedeutung und erleichtert die Einsatzplanung u.a. auch im Hinblick auf die Zuordnung der Rettungskräfte zu den Verletzten als auch die Entscheidung im Hinblick auf die Anforderung weiterer Rettungskräfte und die Anforderung von Krankentransporten, Klinikbetten etc.

Dabei hat es sich als vorteilhaft erwiesen, dass die Identifikationsvorrichtung mit der Sichtungsvorrichtung in einem ausgebildet ist, wobei mindestens ein Zustand der Sichtungsvorrichtung einer bestimmten Berufsgruppe zugeordnet ist.

Des Weiteren ist es erfindungsgemäß vorgesehen, dass die individuelle Kennung der Sichtungsvorrichtung und/oder der Identifikationsvorrichtung zusätzlich mittels eines Daten-Tags, insbesondere eines QR-Codes, Barcodes, Strichcodes, NFC-Tags und/oder Bluetooth, auslesbar ist bzw. ausgelesen wird.

Erfindungsgemäß ist jeder Sichtungsvorrichtung eine individuelle Kennung zugeordnet. Während es aus Sicherheitsgründen insbesondere vorgesehen sein kann, dass die Kennung in Form einer Zahl, oder in Form von Buchstaben, oder einer Kombination von Zahlen und Buchstaben für den Menschen lesbar auf der jeweiligen Sichtungsvorrichtung angebracht ist, ist es besonders vorteilhaft, wenn ein automatisches Auslesen der individuellen Kennung ermöglicht wird. Somit können wichtige Informationen einer Person beispielsweise von einem Notarzt zur Dokumentation durch Auslesen des Daten-Tags zugeordnet werden, ohne dass die Kennung manuell eingegeben werden muss. Dies spart wertvolle Zeit. Auch kann ein Auslesen des Daten-Tags erfolgen um ergänzende Informationen über eine Person in einer Datenbank zu hinterlegen.

Des Weiteren kann das Auslesen eines Daten-Tags und somit der aktuelle Behandlungs- bzw. Transportzustand einer Person automatisch von der Auslesevorrichtung an die Einsatzleitstelle zur Aktualisierung übermittelt werden, so dass nicht nur der Zustand und der Ort einer Person bekannt sind und visualisiert werden können, sondern für die Einsatzplanung auch ersichtlich ist, welche Personen bereits weitergehend versorgt werden. Dies dient unter anderem der Ressourcenallokation, beispielsweise für die Anforderung weiterer Rettungswagen oder von Krankenhausbetten.

Durch ein Auslesen des Datentags wird zudem die Dokumentation des Einsatzes optimiert, da jeder Schritt direkt zentral von dem erfindungsgemäßen System erfassbar ist und visualisiert werden kann.

Gemäß einer Ausführungsform der vorliegenden Erfindung ist es bevorzugt, dass die Sichtungsvorrichtung und/oder die Identifikationsvorrichtung eine Positionserfassungsvorrichtung umfasst, die die Position der Vorrichtung erfasst, insbesondere in Form eines GPS-Empfängers, wobei die Daten der Positionserfassungsvorrichtung mittels der Datenübertragungseinrichtung drahtlos an die Empfangseinrichtung übermittelbar sind bzw. übermittelt werden und/oder das System die Position der Sichtungsvorrichtung und/oder der Identifikationsvorrichtung mittels der Gateways der Empfangseinrichtung trianguliert und/oder ein Indoor-Navigationssystem verwendet wird, insbesondere in Form einer WLAN-Ortung und/oder in Form von Beacons.

Durch eine erfindungsgemäße Positionserfassungsvorrichtung kann ermöglicht werden, dass der Ort einer begutachteten Person automatisch an die Empfangseinheit übermittelt und somit der Einsatzleitstelle zur Kenntnis gebracht wird. Dies hat den Vorteil, dass automatisiert ein geographisch basierter Lageplan erstellt werden kann, der die Anzahl, den Zustand sowie die Position der erfassten Personen in nahezu Echtzeit abbildet.

Auch kann es vorgesehen sein, dass die Sichtungsvorrichtung eine Aktivitätserfassungeinrichtung umfasst, die eine Bewegung der Vorrichtung erfasst, insbesondere eine Beschleunigung, und/oder eine Lage der Vorrichtung, und/oder eine Drehrate der Vorrichtung, wobei die Aktivitätserfassungsvorrichtung insbesondere einen Beschleunigungssensor und/oder einen Lagesensor und/oder einen Drehratensensor und/oder eine integrierte Variante der genannten Sensoren umfasst und/oder einen Lagesensor umfasst und/oder eine Vitalparametererfassungseinrichtung umfasst ist, die ausgelegt und eingerichtet ist, um Vitalparameter einer Person zu erfassen, insbesondere die Herzfrequenz, den Blutdruck, die pulsoxymetrisch gemessene Sauerstoffsättigung (SpO2), die Körpertemperatur und/oder die Atemfrequenz, und wobei die Datenübertragungseinrichtung den eingestellten Zustand, die Position der Sichtungsvorrichtung, eine individuelle Kennung der Vorrichtung, die Beschleunigung der Sichtungsvorrichtung, die Lage der Sichtungsvorrichtung, die Vitalparameter der Person und/oder weitere Daten zum wiederholten Senden an die Empfangseinrichtung in regelmäßigen Zeitabständen überträgt.

Durch eine solche Aktivitätserfassungseinrichtung wird nicht nur der Ort der Personen erkannt, sondern auch der jeweilige Bewegungszustand. Dies hat den Vorteil, dass zentral mittels des erfindungsgemäßen Systems ersichtlich ist, welche Personen sich z.B. bereits eigenständig zu einer Sammelstelle bewegen, welche Personen von Rettungskräften transportiert werden, etc. Auch dies dient der Einsatzplanung und erleichtert dieselbe erheblich.

Die automatische Erfassung der Vitalparameter einer Person kann der Überprüfung der Einschätzung der Erstsichter dienen und insbesondere auch eine Verschlechterung des Zustands einer Person für die Einsatzleitstelle anzeigen. Sofern die Vitalwerte unter bestimmte Schwellwerte gemäß der eingesetzten Klassifikation (bspw. STaRT) fallen, kann zudem eine Warnung erfolgen und/oder eine automatisierte Reklassifizierung der Person durch das erfindungsgemäße System vorgenommen werden.

Dabei kann es insbesondere vorteilhaft sein, dass der eingestellte Zustand einen Zustand einer infolge eines Schadensereignisses verletzten Person repräsentiert, wobei der Zustand eine Kenngröße der Prioritätsstufe zur Versorgung der verletzten Person darstellt, insbesondere gemäß der Triagekategorie T1 bis T4.

Gemäß einer Ausführungsform der vorliegenden Erfindung kann vorgesehen sein, dass die Sichtungsvorrichtung ein Befestigungsmittel zur Befestigung an einer Person umfasst.

Es kann sich erfindungsgemäß als vorteilhaft erweisen, wenn eine Befestigung der Sichtungsvorrichtung mittels eines Befestigungsmittels an der Person erfolgt. Dies kann mittels einer Schlaufe, die um den Hals einer Person gelegt wird, realisiert werden, jedoch auch mit anderen gängigen Befestigungsmitteln wie Armbändern, die fest am Körper anliegen, um eine Erfassung der Vitalparameter zur ermöglichen.

Auch kann bevorzugt sein, dass die Sichtungsvorrichtung wasserdicht ausgebildet ist, wobei insbesondere das Einstellelement in Form eines magnetischen Schiebe- und/oder Drehschalters ausgebildet ist.

Der Zuverlässigkeit der erfindungsgemäßen Sichtungsvorrichtung wird besondere Bedeutung beigemessen. Es hat sich gezeigt, dass in besonders vorteilhafter Weise die Sichtungsvorrichtung selbst wasserdicht ausgebildet ist, um beispielsweise auch bei Unwetter und starkem Regenfall zuverlässig zu funktionieren. Um dies zu ermöglichen, kann es vorteilhaft sein, dass das Einstellelement die eingestellten Werte mittels eines magnetbasierten Dreh- und/oder Schiebeschalters übermittelt, da das Bedienteil des Dreh- und/oder Schiebschalters außerhalb eines gekapselten Gehäuses der Sichtungsvorrichtung angeordnet sind und bedient werden.

Des Weiteren kann es erfindungsgemäß bevorzugt sein, dass das System ausgelegt und eingerichtet ist, um die Position der Sichtungsvorrichtungen und/oder der Identifikationsvorrichtungen, sowie insbesondere den Zustand der Personen und/oder weiterer Informationen bezüglich einer Person auf einer Anzeigeeinrichtung in Form einer Karten- und/oder Listendarstellung anzuzeigen.

Zur schnellen und einfachen Lageeinschätzung hat es sich als vorteilhaft erwiesen, wenn eine grafische Übersicht anzeigbar ist. Dies kann sowohl in Form von Listen als auf grafischen Kartendarsteilungen erfolgen, wobei der Zustand der Personen zudem farblich hervorgehoben werden kann. Auch können bestimmte Berufsgruppen der Einsatzkräfte, die Position von Krankenwagen, etc. und deren Position direkt erkannt und als Entscheidungsgrundlage für die weitere Einsatzplanung herangezogen werden.

Erfindungsgemäß hat es sich zudem als bevorzugt erwiesen, dass die Sichtungsvorrichtung selbst periodisch relevante Daten mittels der Datenübertragungseinrichtung an die Empfangseinrichtung überträgt, ohne dass eine Datenabfrage an die Vorrichtung gerichtet werden muss. Die automatisch ausgesendeten Daten der Sichtungsvorrichtung können dabei von einer Antenne beispielswiese des Long Range Wide Area Network direkt erfasst und weitergeleitet werden

Auch kann es vorgesehen sein, dass das System ausgelegt und eingerichtet ist, um basierend auf den erfassten Informationen der Sichtungsvorrichtungen Anweisungen an mobile Endgeräte zu übersenden, insbesondere den Zustand und die Position einer mittels eines Sichtungsgeräts erfassten Person, wobei die Anweisung manuell oder automatisch versendet werden kann.

Es hat sich als besonders vorteilhaft erwiesen, dass mittels des erfindungsgemäßen Systems direkt Anweisungen an die Einsatzkräfte versandt werden können. Dies kann automatisiert erfolgen, beispielsweise indem die Anzahl kritischer Patienten mit der Anzahl der verfügbaren Rettungswagen abgeglichen wird und im Falle eines Unterschreitens der benötigten Anzahl von Rettungswagen automatisch weitere Rettungswagen angefordert werden. Auch können bestimmten Einsatzkräften bestimmte Aufgaben bzw. Personen zugewiesen werden.

Dabei kann es insbesondere vorteilhaft sein, dass mindestens ein mobiles Endgerät umfasst ist, und die erfassten Positionen der Sichtungsvorrichtungen und/oder der Identifikationsvorrichtungen auf dem mobilen Endgerät anzeigbar bzw. angezeigt sind.

Somit wird nicht nur in der Leitstelle, sondern auch den jeweiligen Einsatzkräften eine grafische Übersicht über die aktuelle Lage ermöglicht. Auch können somit die Einsatzkräfte verletzte Personen mittels der an diesen angeordneten Sichtungsvorrichtungen direkt sehen und zu diesen gelangen.

Des Weiteren liefert die vorliegende Erfindung ein Verfahren zur Ortung und Sichtung bei Schadensereignissen unter Einsatz des Systems gemäß Anspruch 1,
umfassend die folgenden Schritte, insbesondere in dieser Reihenfolge:
a) Bereitstellen von mindestens einer Sichtungsvorrichtung;
b) Aktivieren der Sichtungsvorrichtung;
c) Übertragen der Position und der individuellen Kennung der Sichtungsvorrichtung mittels der Datenübertragungseinrichtung an den Empfänger;
d) Möglichkeit zur Anzeige der Daten an einer Anzeigeeinrichtung;
e) Einstellen des Zustands einer Person an der Sichtungsvorrichtung;
f) Übertragen des Zustands mittels der Datenübertragungseinrichtung an den Empfänger;
g) Bereitstellen der von dem Sichtungsgerät übermittelten Daten an den mindestens einen Server und Anzeigen der Daten auf mindestens einer Anzeigeeinrichtung.

Weiterhin kann ein erfindungsgemäßes Verfahren gemäß einer Ausführungsform die folgenden Schritte umfassen, insbesondere in dieser Reihenfolge
h) Aggregation und Verarbeitung der vom Sichtungsgerät übermittelten Daten;
i) Aufbereitung und Annotation der einsatzrelevanten Daten zu versorgungs- und entscheidungsgrundlegenden Daten; und
j) Bereitstellen der im System auf dem mindestens einen Server vorliegenden Daten auf mobilen Anzeige- und Bearbeitungsgeräten

Gemäß einem erfindungsgemäßen Verfahren werden im Falle eines Massenanfalls von Verletzten zunächst als Erstsichter eingeteilten Rettungskräften von der Einsatzleistelle erfindungsgemäße Sichtungsvorrichtungen bereitgestellt. Diese werden von den Erstsichtern den aufgefundenen Personen zugeteilt und der Zustand der jeweils aufgefundenen Position wird eingestellt.

Der Zustand, sowie insbesondere die Position der aufgefundenen Person wird anschließend automatisch mittels der Datenübertragungseinrichtung der Sichtungsvorrichtung an den Empfänger, und von diesem an den mindestens einen Server übertragen.

Die von dem Server empfangenen Daten werden anschließend auf mindestens einer Anzeigeeinrichtung dargestellt, so dass direkt eine aktuelle Lageübersicht über Anzahl, Ort und Zustand der betroffenen Personen anzeigbar ist.

Bei dem erfindungsgemäßen Verfahren kann insbesondere vorgesehen sein, dass die Anzeige der Daten mittels einer grafischen Visualisierung erfolgt, insbesondere in Form einer Kartendarstellung, wobei jedes Sichtungsgerät in Form eines grafischen Elements dargestellt wird, insbesondere mit einer korrespondierenden farblichen Markierung zu der farblichen Markierung an dem Einstellelement der Sichtungsvorrichtung repräsentativ für einen Zustand der gesichteten Person.

Es hat sich als besonders vorteilhaft erwiesen, dass der Ort, die Anzahl und der Zustand der erfassten Personen auf einer Karte und besonders bevorzugt parallel in Form einer Liste dargestellt sind. Im Falle der Kartendarstellung wird eine möglichst einfache Erfassung des Zustands der jeweiligen Person dadurch erreicht, dass die grafische Repräsentation für die jeweilige Person in der Farbe erfolgt, die zu der eingestellten Farbe an der Sichtungsvorrichtung korrespondiert. Die Einsatzkräfte sind an die bekannte Farben und deren Bedeutung gewöhnt, so dass keine Umstellung erforderlich ist.

Auch ist es bevorzugt, dass das Verfahren die folgenden Schritte umfasst:
k) Bereitstellen von mindestens einer Identifikationsvorrichtung;
l) Zuordnen der Identifikationsvorrichtung zu einer bestimmten Person einer bestimmten Berufsgruppe;
m) Anzeigen der Position der Identifikationsvorrichtung und insbesondere gleichzeitige Anzeige der Berufsgruppe auf der Anzeigeeinrichtung, insbesondere zusätzlich zu der Anzeige der Sichtungsvorrichtungen.

Es hat sich parallel zur Sichtung der verletzten Personen als vorteilhaft erwiesen, wenn eine grafische Darstellung der Berufsgruppe und der Position der sich jeweils im Einsatz befindlichen Rettungskräfte erfolgt.

Dabei kann insbesondere vorgesehen sein, dass jeder Identifikationsvorrichtung ein bestimmter räumlicher Abschnitt zugewiesen wird, wobei der Abschnitt auf der Anzeigeeinrichtung und/oder einer Anzeigeeinrichtung eines mobilen Endgeräts anzeigbar ist, so dass bestimmte räumliche Abschnitte bestimmten Personen zuordbar sind.

Dies hat den Vorteil, dass die meist unzureichend verfügbaren Einsatzkräfte bestmöglich im Schadensgebiet verteilt werden können, ohne dass jede Einsatzkraft individuell informiert werden muss. Insbesondere wird hierdurch vermieden, dass viele Einsatzkräfte sich in bestimmten Gebieten konzentrieren, während andere Bereiche ausgespart bleiben.

Auch kann es vorteilhaft sein, dass die mittels der Identifikationsvorrichtung erfassten Personen den Einsatzkräften, insbesondere Notärzten, Sanitätern, Rettungswagen, durch eine Eingabe am Server zugeordnet werden, bevorzugt mittels Drag and Drop, und die jeweiligen Einsatzkräfte automatisch mittels einer Benachrichtigung über die Zuordnung informiert werden.

Dies stellt eine signifikante Vereinfachung der Planung und Steuerung des Einsatzes dar.

Dabei kann gemäß einer Ausführungsform der vorliegenden Erfindung auch vorgesehen sein, dass der Daten-Tag der Sichtungsvorrichtung von einem mobilen Endgerät ausgelesen wird und zusätzliche Informationen über die gesichtete Person an dem mobilen Endgerät eingegeben und dem Server bereitgestellt werden.

Dies dient zum einen der durchgehenden elektronischen Dokumentation, gleichsam aber auch der Aktualisierung des Einsatzes. Beispielsweise kann ein Notarzt weitere Zustandsinformationen eingeben, so dass ein Rettungshubschrauber automatisiert oder manuell angefordert oder bestimmte Einrichtungen in Krankenhäusern und Behandlungsverfahren vorbereitet werden, indem das erfindungsgemäße System die entsprechenden Informationen den Krankenhäusern und weiteren Systemen bereitstellt.

Schließlich kann vorgesehen sein, dass eine grafische und/oder tabellarische Übersicht über die Sichtungsvorrichtungen und/oder die Identifikationsvorrichtungen sowie mindestens eines Teils der von den Sichtungsvorrichtungen und/oder die Identifikationsvorrichtungen an den mindestens einen Server übermittelten Informationen über den Zustand der mit den jeweiligen Vorrichtungen zugeordneten Personen erfolgt.

## Patentansprüche

1. System zum Orten, Sichten, Klassifizieren, Verwalten und Koordinieren von verletzten, betroffenen oder kontaminierten Personen, sowie für schnelle und präzise Übersicht über die Anzahl der betroffenen Personen im Falle eines Massenanfalls von Verletzten in Echtzeit und eine optimierte Allokation der begrenzten Ressourcen, sowie für eine Zuordnung der erfassten Personen auf Einsatzkräfte, insbesondere Notärzte, Sanitäter oder Rettungswagen, sowie zum Orten und Verwalten von Helfern, wobei das System mindestens zwei am Körper von Personen getragene mobile Sichtungsvorrichtungen, mindestens eine Empfangseinrichtung, mindestens einen Server und mindestens eine Anzeige- und Interaktionseinrichtung umfasst, wobei jede Sichtungsvorrichtung ein Einstellelement zum Einstellen von mindestens zwei unterschiedlichen Zuständen einer Person, eine, insbesondere feste, individuelle Kennung und eine Datenübertragungseinrichtung umfasst, wobei der eingestellte Zustand sowie die individuelle Kennung mittels der Datenübertragungseinrichtung, insbesondere in Echtzeit, drahtlos an die Empfangseinrichtung übermittelbar ist bzw. übermittelt wird, wobei die Kennung zusätzlich mittels eines Daten-Tags auslesbar ist bzw. ausgelesen wird, wobei der mindestens eine Server mittels der von der mindestens einen Empfangseinrichtung empfangenen Daten die Anzahl und den Zustand von Personen erfasst und auf einer Anzeigeeinrichtung anzeigt bzw. einem Interaktionsmedium bereitstellt, in dem die Informationen verarbeitet, aggregiert und aufbereitet werden, und wobei die Anzeige- und Interaktionseinrichtungen sowohl stationär als auch mobil realisiert sein können, und wobei die drahtlose Kommunikation der Datenübertragungseinrichtung mit der Empfangseinrichtung über eine Long range, low power wireless platform oder ein Long Range Wide Area Network erfolgt, wobei insbesondere die Sichtungsvorrichtungen mit Gateways als Empfangseinrichtungen kommunizieren und die Gateways die Datenpakete an den mindestens einen Server senden,
**dadurch gekennzeichnet, dass**
das Einstellelement in Form eines Drehschalters und eines zusätzlichen Schiebeschalters ausgebildet ist, wobei der Drehschalter für jeden Zustand eine von den anderen Zuständen unterscheidbare grafische Kennzeichnung aufweist und durch den Schiebeschalter eine, insbesondere zweistufige, Priorisierung des mit dem Drehschalter eingestellten Zustands einstellbar ist.

2. System nach Anspruch 1, **dadurch gekennzeichnet, dass**
das System mehr als einen Server umfassen kann, wobei die Server miteinander synchronisiert sind, so dass insbesondere ein redundantes System ausgebildet wird, wobei sich die Server insbesondere automatisch synchronisieren und keine manuelle Eingabe oder Interaktion eines Benutzers notwendig ist.

3. System nach einem der vorangehenden Ansprüche, **dadurch gekennzeichnet, dass** mindestens eine Identifikationsvorrichtung umfasst ist, so dass eine Person eindeutig identifiziert oder identifizierbar ist, wobei die Identifikationsvorrichtung eine weitere Datenübertragungseinrichtung umfasst, um mit der Empfangseinrichtung zu kommunizieren, wobei gegebenenfalls die Identifikationsvorrichtung mit der Sichtungsvorrichtung in einem ausgebildet ist, wobei mindestens ein Zustand der Sichtungsvorrichtung einer bestimmten Berufsgruppe zugeordnet ist.

4. System nach einem der vorangehenden Ansprüche, **dadurch gekennzeichnet, dass** die individuelle Kennung der Sichtungsvorrichtung und/oder der Identifikationsvorrichtung zusätzlich mittels eines Daten-Tags, insbesondere eines QR-Codes, Barcodes, Strichcodes, NFC-Tags und/oder Bluetooth, identifizierbar ist.

5. System nach einem der vorangehenden Ansprüche, **dadurch gekennzeichnet, dass** das System ausgelegt und eingerichtet ist, um die Position der Sichtungsvorrichtungen und/oder der Identifikationsvorrichtungen, sowie insbesondere den Zustand der Personen und/oder weiterer Informationen bezüglich einer Person auf einer Anzeigeeinrichtung in Form einer grafischen Kartendarstellung, grafischen Statistikdarstellung, listenbasierten oder textuellen Darstellung anzuzeigen.

6. System nach einem der vorangehenden Ansprüche, **dadurch gekennzeichnet, dass** das System ausgelegt und eingerichtet ist, um basierend auf den erfassten Informationen der Sichtungsvorrichtungen eine Lage zu erfassen, organisieren, koordinieren, sowie Informationen und Anweisungen an mobile Endgeräte zu übersenden, insbesondere den Zustand und die Position einer mittels eines Sichtungsgeräts erfassten Person, wobei die Information und Anweisung manuell oder automatisch versendet werden kann.

7. System nach einem der vorangehenden Ansprüche, **dadurch gekennzeichnet, dass** mindestens ein mobiles Endgerät umfasst ist, und die erfassten Positionen der Sichtungsvorrichtungen und/oder der Identifikationsvorrichtungen auf dem mobilen Endgerät anzeigbar bzw. angezeigt sind.

8. System nach einem der vorangehenden Ansprüche, **dadurch gekennzeichnet, dass** mindestens ein stationäres oder mobiles Endgerät zur Darstellung, Bearbeitung, Erweiterung und Verwaltung der Daten umfasst ist.

9. System nach einem der vorangehenden Ansprüche, **dadurch gekennzeichnet, dass** die mobilen Endgeräte offline arbeiten können und sich nach Sichtbarkeit eines oder mehrerer Server im Netz automatisch synchronisieren.

10. System nach einem der vorangehenden Ansprüche, **dadurch gekennzeichnet, dass** die Server sich datentechnisch automatisch synchronisieren, auch wenn parallel gleiche Einsätze unabhängig auf mindestens zwei bislang nicht miteinander verbundenen und/oder vernetzten Systemen geführt werden, wobei gegebenenfalls
die mindestens zwei verschiedenen Systeme unterschiedlichen Organisationen zugeordnet sind,
oder
die Anzeige- und Interaktionseinrichtungen Benutzergruppen zugeordnet sind, ebenso wie Patienten, wobei durch einen Zugriff und Filter eine automatisierte bzw. lageunterstützte Verwaltung und Koordination erzeugt wird,
oder
Patienten zur Lagebewältigung durch Transportauftragsverwaltung an Transportmittel und Transportziele allokiert werden, wobei Transportmittel insbesondere speziell für Patienten ausgelegte Fahrzeuge bzw. Hubschrauber sind und wobei vorzugsweise Transportziele für die Versorgung der Patienten vorgehaltene Einrichtungen, wie Krankenhäuser, sind,
oder
Transportaufträge aus der Transportauftragsverwaltung automatisch sowie manuell gedruckt und an das Einsatzpersonal zur Bearbeitung übergeben werden können, so dass die Transportauftragsverwaltung die Transportauftragserstellung durch einsatztaktische und patientenbezogene Filterung und Sortierung unterstützt, um die Einsatzbearbeitung priorisiert nach Sichtungsschema durchzuführen,
oder
nachgelagerte Einsatzzentralen, wie integrierte Leitstellen, Feuerwehreinsatzzentralen, bei verfügbarer Infrastruktur mit Daten von der Schadenslage zur übergreifenden Schadensbewältigungs-Zuarbeit von dem System beliefert werden können,
oder
Transportmittel zum Abtransport der Patienten automatisiert mittels der mobilen Endgeräte erfasst und dessen einsatztaktischen Daten, insbesondere dessen Einsatzwert, im System verteilt werden,
oder
Transportziele mit deren Aufnahmekapazitäten, Entfernung zur Schadenslage und Kompatibilität zum Transportmittel (Hubschrauberlandeplatz) vorgehalten, verwaltet und aktualisiert werden.

11. Verfahren zur Ortung, Sichtung, Klassifikation, Verwaltung und Koordination von verletzten, betroffenen oder kontaminierten Personen, sowie für schnelle und präzise Übersicht über die Anzahl der betroffenen Personen im Falle eines Massenanfalls von Verletzten in Echtzeit und eine optimierte Allokation der begrenzten Ressourcen, sowie für eine Zuordnung der erfassten Personen auf Einsatzkräfte, insbesondere Notärzte, Sanitäter oder Rettungswagen, sowie zum Orten und Verwalten von Helfern, , unter Verwendung eines Systems nach Anspruch 1, umfassend die folgenden Schritte, insbesondere in dieser Reihenfolge:
a) Bereitstellen von mindestens einer Sichtungsvorrichtung;
b) Aktivieren der Sichtungsvorrichtung;
c) Übertragen der Position und der individuellen Kennung der Sichtungsvorrichtung mittels der Datenübertragungseinrichtung an eine Empfangseinrichtung;
d) Möglichkeit zur Anzeige der Daten an einer Anzeigeeinrichtung;
e) Einstellen des Zustands einer Person an der Sichtungsvorrichtung;
f) Übertragen des Zustands mittels einer Datenübertragungseinrichtung an die Empfangseinrichtung;
g) Bereitstellen der von dem Sichtungsgerät übermittelten Daten an den mindestens einen Server und Anzeigen der Daten auf mindestens einer Anzeigeeinrichtung.

12. Verfahren nach Anspruch 11, **dadurch gekennzeichnet, dass** die Anzeige der Daten mittels einer grafischen Visualisierung, textbasierten oder listenbasierten Darstellung erfolgt, insbesondere in Form einer Kartendarstellung, wobei jedes Sichtungsgerät in Form eines grafischen Elements dargestellt wird, insbesondere mit einer korrespondierenden farblichen Markierung zu der farblichen Markierung an dem Einstellelement der Sichtungsvorrichtung repräsentativ für einen Zustand der Person.

13. Verfahren nach einem der Ansprüche 11 oder 12, weiterhin umfassend die folgenden Schritte:
h) Bereitstellen von mindestens einer Identifikationsvorrichtung;
i) Zuordnen der Identifikationsvorrichtung zu einer bestimmten Person;
j) Anzeigen der Position der Identifikationsvorrichtung und insbesondere gleichzeitige Anzeige der Berufsgruppe auf der Anzeigeeinrichtung, insbesondere zusätzlich zu der Anzeige der Sichtungsvorrichtungen.

## Claims

1. System for locating, viewing, classifying, managing and coordinating injured, affected or contaminated persons, as well as for a quick and precise overview of the number of affected persons in the event of a mass casualty incident in real time and an optimized allocation of limited resources, as well as for an assignment of detected persons to emergency services, in particular emergency physicians, paramedics or ambulances, as well as for locating and managing helpers, wherein the system comprises at least two mobile screening apparatuses carried on the body of persons, at least one receiving device, at least one server and at least one display and interaction device, wherein each screening apparatus comprises a setting element for setting at least two different states of a person, an, in particular fixed, individual identifier and a data transmission device, wherein the set state and the individual identifier can be transmitted wirelessly to the receiving device by means of the data transmission device, in particular in real time, or is transmitted to the receiving device, wherein the identifier is additionally transmitted to the receiving device by means of the data transmission device. wherein the identifier can additionally be or is read out by means of a data tag, wherein the at least one server records the number and the state of persons by means of the data received from the at least one receiving device and displays them on a display device or provides them to an interaction medium in which the information is processed, aggregated and prepared, and wherein the display and interaction devices can be realized both stationary and mobile, and wherein the wireless communication of the data transmission device with the receiving device takes place via a Long range, low power wireless platform or a Long Range Wide Area Network, wherein in particular the display devices communicate with gateways as receiving devices and the gateways send the data packets to the at least one server,
**characterized in that**
the setting element is designed in the form of a rotary switch and an additional slide switch, wherein the rotary switch has a graphic identification for each state which can be distinguished from the other states and the slide switch can be used to set a prioritization, in particular a two-stage prioritization, of the state set with the rotary switch.

2. System according to claim 1, **characterized in that**
the system can comprise more than one server, wherein the servers are synchronized with one another, so that in particular a redundant system is formed, wherein the servers synchronize in particular automatically and no manual input or interaction of a user is necessary.

3. System according to one of the preceding claims, **characterized in that**
at least one identification device is included, so that a person is uniquely identified or identifiable, wherein the identification device includes a further data transmission device to communicate with the receiving device, wherein optionally the identification device is formed in one with the viewing device, at least one state of the viewing device being assigned to a specific occupational group.

4. System according to one of the preceding claims, **characterized in that**
the individual identifier of the viewing device and/or the identification device can additionally be identified by means of a data tag, in particular a QR code, barcode, NFC tag and/or via Bluetooth.

5. System according to one of the preceding claims, **characterized in that**
the system is designed and set up to display the position of the screening apparatuses and/or the identification devices, and in particular the status of the persons and/or further information relating to a person on a display device in the form of a graphical map display, graphical statistics display, list-based or textual display.

6. System according to one of the preceding claims, **characterized in that**
the system is designed and set up to detect, organize, coordinate and send information and instructions to mobile terminals based on the information detected by the screening apparatuses, in particular the status and position of a person detected by means of a screening apparatus, wherein information and instructions can be sent manually or automatically.

7. System according to one of the preceding claims, **characterized in that**
at least one mobile terminal device is included, and the detected positions of the screening apparatuses and/or the identification devices can be displayed or are displayed on the mobile terminal device.

8. System according to one of the preceding claims, **characterized in that**
at least one stationary or mobile terminal device for displaying, processing, expanding and managing the data is included.

9. System according to one of the preceding claims, **characterized in that**
the mobile terminals can work offline and synchronize automatically after visibility of one or more servers in the network.

10. System according to one of the preceding claims, **characterized in that**
the servers synchronize automatically in terms of data technology, even if identical operations are carried out independently in parallel on at least two systems which have not previously been connected and/or networked with one another, where appropriate the at least two different systems are assigned to different organizations,
or
the display and interaction devices are assigned to user groups, just like patients, whereby automated or position-supported administration and coordination is generated by means of an access and filter,
or
patients, for situation management, are allocated to means of transport and transport destinations by transport order management, wherein means of transport are in particular vehicles or helicopters specially designed for patients and wherein preferably transport destinations are facilities provided for the care of patients, such as hospitals,
or
transport orders from the transport order management system can be printed automatically and manually and transferred to the emergency personnel for processing, so that the transport order management system supports the creation of transport orders by means of tactical and patient-related filtering and sorting in order to prioritize the processing of operations according to the viewing scheme,
or
downstream operations centers, such as integrated control centers, fire department operations centers, can be supplied with data from the damage situation for comprehensive damage management work by the system if the infrastructure is available,
or
means of transportation for the removal of patients are automatically recorded by the mobile terminals and their tactical data, in particular their operational value, are distributed in the system,
or
transport destinations with their reception capacities, distance to the damage situation and compatibility with the means of transport (helipad) are stored, managed and updated.

11. Method for locating, viewing, classifying, managing and coordinating injured, affected or contaminated persons, as well as for a quick and precise overview of the number of affected persons in the event of a mass casualty incident in real time and an optimized allocation of limited resources, as well as for an assignment of the detected persons to emergency personnel, in particular emergency physicians, paramedics or ambulances, as well as for locating and managing helpers, using a system according to claim 1, comprising the following steps, in particular in this order:
a) providing at least one screening apparatus;
b) activating the screening apparatus;
c) transmitting the position and the individual identifier of the screening apparatus to a receiving device by means of the data transmission device;
d) possibility of displaying the data on a display device;
e) setting the status of a person on the screening apparatus;
f) transmitting the status to the receiving device by means of a data transmission device;
g) providing the data transmitted by the screening apparatus to the at least one server and displaying the data on at least one display device.

12. Method according to claim 11, **characterized in that**
the data is displayed by means of a graphical visualization, text-based or list-based representation, in particular in the form of a map representation, each screening apparatus being represented in the form of a graphical element, in particular with a corresponding coloured marking to the coloured marking on the setting element of the screening apparatus representative of a state of the person.

13. Method according to any one of claims 11 or 12, further comprising the following steps:
h) providing at least one identification device;
i) associating the identification device with a particular person;
j) displaying the position of the identification device and, in particular, simultaneously displaying the occupational group on the display device, in particular in addition to displaying the screening apparatuses.

## Revendications

1. Système de localisation, de triage, de classification, de gestion et de coordination de personnes blessées, affectées ou contaminées, ainsi que pour une vue d'ensemble rapide et précise du nombre des personnes affectées en cas d'afflux massif de blessés en temps réel et pour une allocation optimisée des ressources limitées, ainsi que pour une affectation des personnes détectées aux forces d'intervention, notamment aux médecins urgentistes, aux secouristes ou aux ambulances, ainsi que pour localiser et gérer les sauveteurs, le système comprenant au moins deux dispositifs de triage mobiles portés par les personnes, au moins un équipement de réception, au moins un serveur et au moins un équipement d'affichage et d'interaction, chaque dispositif de triage comprenant un élément de réglage permettant de régler au moins deux états différents d'une personne, un identifiant individuel, notamment fixe, et un équipement de transmission de données, l'état réglé ainsi que l'identifiant individuel pouvant être transmis sans fil à l'équipement de réception, notamment en temps réel, au moyen de l'équipement de transmission de données, l'identifiant étant en outre lisible ou lu au moyen d'une étiquette de données, ledit au moins un serveur détectant le nombre et l'état des personnes au moyen des données reçues par ledit au moins un équipement de réception et les affichant sur un équipement d'affichage ou les mettant à disposition sur un support d'interaction dans lequel les informations sont traitées, agrégées et préparées, et les équipements d'affichage et d'interaction pouvant être réalisés aussi bien sous forme stationnaire que mobile,
et la communication sans fil du dispositif de transmission de données avec le dispositif de réception étant effectuée par l'intermédiaire d'une plate-forme sans fil longue portée et basse puissance ou d'un réseau longue portée étendu, les dispositifs de triage communiquant notamment avec des passerelles en tant en tant qu'équipements de réception et les passerelles envoyant les paquets de données audit au moins un serveur, **caractérisé en ce que**
l'élément de réglage est conçu sous la forme d'un commutateur rotatif et d'un commutateur à glissière supplémentaire, le commutateur rotatif présentant pour chaque état un marquage graphique différenciable des autres états et le commutateur à glissière permettant de régler une priorité, notamment à deux niveaux, de l'état réglé à l'aide du commutateur rotatif.

2. Système selon la revendication 1, **caractérisé en ce que**
le système peut comprendre plus d'un serveur, les serveurs étant synchronisés les uns avec les autres de manière à réaliser en particulier un système redondant, les serveurs se synchronisant notamment automatiquement et aucune entrée ou interaction manuelle d'un utilisateur n'étant nécessaire.

3. Système selon l'une quelconque des revendications précédentes, **caractérisé en ce qu'**il est prévu
au moins un dispositif d'identification de telle sorte qu'une personne soit identifié ou identifiable sans ambiguïté, le dispositif d'identification comprenant un autre équipement de transmission de données pour communiquer avec le dispositif de réception, dans lequel, le cas échéant le dispositif d'identification est réalisé en un seul dispositif avec le dispositif de triage, au moins un état du dispositif de triage étant associé à un groupe professionnel déterminé.

4. Système selon l'une quelconque des revendications précédentes, **caractérisé en ce que**
l'identifiant individuel du dispositif de triage et/ou du dispositif d'identification peut être identifié de manière supplémentaire au moyen d'une étiquette de données, en particulier d'un code QR, d'un code à barres, d'une étiquette NFC et/ou du Bluetooth.

5. Système selon l'une quelconque des revendications précédentes, **caractérisé en ce qu'**il est prévu
le système est configuré et conçu pour afficher la position des dispositifs de triage et/ou des dispositifs d'identification, ainsi que, notamment, l'état des personnes et/ou d'autres informations concernant une personne sur un équipement d'affichage sous la forme d'une représentation de carte graphique, d'une représentation statistique graphique, d'une représentation à base de listes ou textuelle.

6. Système selon l'une quelconque des revendications précédentes, **caractérisé en ce qu'**il est prévu
le système est configuré et conçu pour détecter, organiser et coordonner une situation sur la base des informations détectées par les dispositifs de triage, ainsi que pour transmettre des informations et des instructions à des terminaux mobiles, notamment l'état et la position d'une personne détectée au moyen d'un appareil de triage, les informations et les instructions pouvant être envoyées manuellement ou automatiquement.

7. Système selon l'une quelconque des revendications précédentes, **caractérisé en ce qu'**il est prévu
au moins un terminal mobile et **en ce que** les positions détectées des dispositifs de triage et/ou des dispositifs d'identification peuvent être affichées ou sont affichées sur le terminal mobile.

8. Système selon l'une quelconque des revendications précédentes, **caractérisé en ce qu'**il est prévu
au moins un terminal fixe ou mobile pour représenter, traiter, compléter et gérer les données.

9. Système selon l'une quelconque des revendications précédentes, **caractérisé en ce que**
les terminaux mobiles peuvent fonctionner hors ligne et se synchroniser automatiquement après qu'un ou plusieurs serveurs sont devenus visibles sur le réseau.

10. Système selon l'une quelconque des revendications précédentes, **caractérisé en ce que**
les serveurs se synchronisent automatiquement en ce qui concerne les données, même si des interventions identiques sont menées en parallèle et indépendamment sur au moins deux systèmes qui n'étaient pas jusqu'alors connectés et/ou mis en réseau les uns avec les autres, dans lequel, le cas échéant lesdits au moins deux systèmes différents sont associés à des organisations différentes,
ou
les équipements d'affichage et d'interaction sont affectés à des groupes d'utilisateurs, ainsi qu'à des patients, un accès et un filtre générant une gestion et une coordination automatisées ou assistées par la position,
ou
les patients sont alloués à des moyens de transport et à des destinations de transport par la gestion des ordres de transport à des fins de gestion de la situation, les moyens de transport étant notamment des véhicules ou des hélicoptères spécialement adaptés aux patients et les destinations de transport étant de préférence des établissements réservés à la prise en charge des patients, tels que des hôpitaux,
ou
des ordres de transport peuvent être imprimés automatiquement ou manuellement à partir de la gestion des ordres de transport et transmis au personnel d'intervention pour traitement, de telle sorte que la gestion des ordres de transport aide à l'établissement des ordres de transport par un filtrage et un triage tactiques d'intervention et axés sur le patient, afin de mettre en œuvre le traitement des interventions par ordre de priorité selon un schéma de triage,
ou
des centrales d'intervention situées en aval, telles que des postes de commandement intégrés, des centrales d'intervention des pompiers, peuvent, si l'infrastructure le permet, recevoir en provenance du système des données concernant le lieu de l'accident afin de pouvoir fournir une assistance globale à la gestion des dommages,
ou
des moyens de transport destinés à évacuer les patients sont détectés automatiquement au moyen des terminaux mobiles et leurs données tactiques d'intervention, notamment leur valeur d'intervention, sont réparties dans le système,
ou
des destinations de transport avec leurs capacités d'accueil, la distance par rapport au lieu de l'accident et la compatibilité avec le moyen de transport (héliport) sont conservées, gérées et mises à jour.

11. Procédé de localisation, de triage, de classification, de gestion et de coordination de personnes blessées, affectées ou contaminées, ainsi que pour une vue d'ensemble rapide et précise du nombre de personnes affectées en cas d'afflux massif de blessés en temps réel et une allocation optimisée des ressources limitées, ainsi que pour une affectation des personnes détectées aux forces d'intervention, notamment aux médecins urgentistes, aux secouristes ou aux ambulances, ainsi que pour localiser et gérer les sauveteurs, à l'aide d'un système selon la revendication 1, comprenant les étapes suivantes, en particulier dans cet ordre :
a) la mise à disposition d'au moins un dispositif de triage ;
b) l'activation du dispositif de triage ;
c) la transmission de la position et de l'identifiant individuel du dispositif de triage à un équipement de réception au moyen d'un équipement de transmission de données ;
d) la possibilité d'afficher les données sur un équipement d'affichage ;
e) le réglage de l'état d'une personne sur le dispositif de triage ;
f) la transmission de l'état au moyen d'un équipement de transmission de données à l'équipement de réception ;
g) la mise à disposition des données transmises par l'appareil de triage audit au moins un serveur et l'affichage des données sur au moins un équipement d'affichage.

12. Procédé selon la revendication 11, **caractérisé en ce que**
l'affichage des données est effectué au moyen d'une visualisation graphique, d'une représentation textuelle ou à base de listes, notamment sous forme d'une représentation de carte, chaque appareil de triage étant représenté sous forme d'un élément graphique, notamment à l'aide d'un marquage coloré correspondant au marquage coloré sur l'élément de réglage du dispositif de triage, représentatif d'un état de la personne.

13. Procédé selon l'une quelconque des revendications 11 ou 12, comprenant en outre les étapes suivantes :
h) la mise à disposition d'au moins un dispositif d'identification ;
i) l'affectation du dispositif d'identification à une personne déterminée ;
j) l'affichage de la position du dispositif d'identification et notamment l'affichage simultané du groupe professionnel sur l'équipement d'affichage, notamment en plus de l'affichage des dispositifs de triage.
